# EUROPEAN PATENT SPECIFICATION

(11) **EP 0 586 545 B1**
(45) Date of publication and mention of the grant of the patent: **05.03.1997**
(21) Application number: 92912579.7
(22) Date of filing: 01.05.1992
(51) Int. Cl.: G02F 1/167, G09G 3/34

(54) **ELECTROPHORETIC DISPLAY PANEL WITH TAPERED GRID INSULATORS AND ASSOCIATED METHODS**
ELEKTROPHORETISCHE ANZEIGEVORRICHTUNG MIT ABGESCHRAEGTEN GITTERISOLATOREN UND DAMIT VERBUNDENE VERFAHREN
PANNEAU D'AFFICHAGE ELECTROPHORETIQUE POURVU D'ISOLATEURS DE GRILLE A EXTREMITE AMINCIE ET PROCEDES ASSOCIES

(30) Priority: 06.05.1991 US 696169
(43) Date of publication of application: 16.03.1994
(73) Proprietor: COPYTELE INC., Huntington Station New York 11746 (US)
(72) Inventor: DISANTO, Frank, J., North Hills, NY 11030 (US); KRUSOS, Denis, A., Lloyd Harbor, NY 11743 (US)
(74) Representative: Lucking, David John
(86) International application number: US9203654
(87) International publication number: WO9220060

(56) References cited:
- US-A- 4 742 345
- US-A- 4 766 426
- US-A- 4 853 296
- US-A- 5 006 212

## Description

### Technical Field of the Invention

The present invention relates to electrophoretic display panel apparatus and methods for fabricating same, and more particularly, to a triode-type electrophoretic display panel having an improved grid insulator configuration permitting more efficient electrical connection to display driver circuitry.

### Background Art

A variety of electrophoretic display panels are now known. Of most direct pertinence to the present invention are those shown and described in U.S. Patent No. 4,655,897 entitled "Electrophoretic Display Panels and Associated Methods", U.S. Patent No. 4,742,345, entitled "Electrophoretic Display Panel Apparatus and Methods Therefor", U.S. Patent No. 4,772,820 entitled 'Monolithic Flat Panel Display Apparatus" and WO 92/15982 (U.S. Patent Application No. 667,630 entitled ELECTROPHORETIC DISPLAY PANEL WITH PLURAL ELECTRICALLY INDEPENDENT ANODE ELEMENTS, (CopyTele 3.0-17) filed on March 11, 1991). Each of the foregoing U.S. Patents as well as the application referred to, is in the name of Frank J. DiSanto and Denis A. Krusos, the inventors herein, and are assigned to the assignee herein, Copytele, Inc. The display panels shown in the foregoing patents and application operate upon the same basic principle, viz., if a suspension of electrically charged pigment particles in a dielectric fluid is subjected to an applied electrostatic field, the pigment particles will migrate through the fluid in response to the electrostatic field. Given a substantially homogeneous suspension of particles having a pigment color different from that of the dielectric fluid, if the applied electrostatic field is localized it will cause a visually observable localized pigment particle migration. The localized pigment particle migration results either in a localized area of concentration or rarefaction of particles depending upon the sign and direction of the electrostatic force and the charge on the pigment particles. The electrophoretic display apparatus taught in each of the foregoing U.S. Patents are triode type displays having a plurality of independent, parallel cathode conductor members deposited in the horizontal on one surface of a glass viewing screen. A layer of insulating photoresist material deposited over the cathode members and photoetched down to the cathode members to yield a plurality of insulator strips positioned at right angles to the cathode members, forms the substrate for a plurality of independent, parallel grid conductor members running in the vertical direction. A glass cap member forms a fluid-tight seal with the viewing window along the cap's peripheral edge for containing the fluid suspension and also acts as a substrate for the anode which is a conductor layer deposited on the interior flat surface of the cap. The term "cap" is used in its broadest sense as the cap member may include a glass sheet used as the anode with a peripheral seal joining the anode to the grid-cathode assembly. Such structure is seen in the above-noted patents. When the cap is in place, the anode surface is in spaced parallel relation to both the cathode members and the grid members. Given a specific particulate suspension, the sign of the electrostatic charge which will attract and repel the pigment particles will be known. The cathode member voltage and the grid member voltage can then be ascertained such that when a particular voltage is applied to the cathode and another voltage is applied to the grid, the area proximate their intersection will assume a net charge sufficient to attract or repel pigment particles in suspension in the dielectric fluid. Since numerous cathode and grid lines are employed, there are numerous discrete intersection points which can be controlled by varying the voltage on the cathode and grid members to cause localized visible regions of pigment concentration and rarefaction. Essentially then, the operating voltages on both cathode and grid must be able to assume at least two states corresponding to a logical one and a logical zero. Logical one for the cathode may either correspond to attraction or repulsion of pigment. Typically, the cathode and grid voltages are selected such that only when a first voltage difference appears at a particular intersection point, then a sufficient electrostatic field will be present at the intersection to cause the writing of a visual bit of information on the display. The bit may be erased when a second state occurs. In this manner, digitized data can be displayed on the electrophoretic display.

The electrophoretic displays described above utilize numerous electrically and physically independent cathode and grid members. For example, an 8 1/2" X 11" display screen with a resolution of 200 lines per inch has 2,200 horizontal cathode row members and 1,700 vertical column grid members. In general, it is desirable to have the greatest number of horizontal and vertical members with the smallest possible width. This results in increased resolution and screen brightness, i.e., the more coordinates, the greater the resolution, the smaller the width of each element, the less the electrophoretic effect is obscured. Thus, the electrophoretic display raises a technical challenge that is common in the field of densely-packed miniaturized electrical devices, viz., while it is possible, using photoetching techniques and the like, to create extremely small circuit components, it is sometimes difficult to make the numerous electrical connections necessary to integrate the miniature components, in this case, the cathode and grid members and the display drivers, into a circuit. A variety of techniques to facilitate connection of miniature components have been developed. For example, U.S. Patent No. 4,772,820 teaches an improved means for connecting numerous miniature cathode and grid members to display drivers. In accordance with that patent, the ends of the cathode and grid members resident upon the surface of the glass viewing screen of the display are metalized and grouped into a pattern which is adapted to electrically connect to mating output contacts of a driver circuit that is bonded to the screen at a predetermined aligned location. The bonding of the respective mating contacts is performed using wire bonding techniques which can be automated to yield quick and efficient connections. In yet a further aspect of the '820 patent, the inputs to the driver circuit are also wire bonded to patterned input conductors provided on the surface of the screen, thus yielding a substantially monolithic display screen having integral associated driver circuits.

Both U.S. Patent No. 4,742,345 and 4,772,820 utilize a grid comprised of numerous electrically and physically independent elements which supply the horizontal coordinate for each displayable location. The grid elements in the foregoing patents are spaced away from the cathode elements by an insulation layer.

U.S. Patent No. 4,772,820 teaches that a connection between the grid elements on one plane and screen surface resident driver circuitry may be made by wire bonding, wherein a wire segment traverses the distance between, and is bonded at either end to, a grid element and a driver circuit terminal. This wire bonding technique is compromised, however, in that the photoresist layer underlying the grid elements is deformable under the pressure of the wire bonding machine and does not, therefore, make an optimal bond either in terms of strength of bond or ease of making it.

The Application cited above, on the other hand, sets forth an alternative display structure utilizing an electrically equipotential grid having only a single electrical connection and an anode with a plurality of discrete elements. While the device taught in the aforementioned application is certainly a solution to the connection problem, the present invention is directed to displays having a grid comprised of multiple discrete elements rather than a monolithic or monopotential grid.

It is therefore an object of the present invention to provide an electrophoretic display which provides an improved conductor pathway from the plane of the grid to that of the viewing screen whereon driver circuitry resides.

It is a further object to provide a stable base to expedite wirebonding of display elements to display circuitry.

It is yet another objective to provide an electrophoretic display which is easier and more economical to produce by simplifying the connection of the numerous vertical display elements to their respective driver circuits.

### Disclosure of the Invention

The problems and disadvantages associated with conventional electrophoretic displays are overcome by the electrophoretic display defined in claim 1 and the method of making such a display as defined in claim 12. The electrophoretic display of the present invention includes a fluid-tight envelope for containing electrophoretic fluid and having a planar portion thereof which is at least partially transparent. The fluid has pigmented particles suspended therein. A plurality of elongated substantially parallel horizontal conductor members are deposited upon the planar portion and contained within the envelope, along with a plurality of elongated substantially parallel vertical conductor members. A plurality of elongated insulator strips are interposed between and electrically insulate the horizontal members from the vertical members, the strips being affixed to both. Each of the strips support a corresponding vertical member thereon a selected distance from the horizontal members and tapers at least one end thereof such that the vertical member contacts the planar portion proximate the tapered end. The horizontal and vertical members form a matrix with a plurality of intersections, each of the members being selectively electrically chargeable to induce movement of the particles within the fluid. The particles are at least partially visible through the planar portion of the envelope.

Further embodiments of the invention are defined in the dependent claims.

### Brief Description of the Drawings

For a better understanding of the present invention, reference is made to the following detailed description of an exemplary embodiment considered in conjunction with the accompanying drawings, in which:

FIG. 1 is an exploded perspective view of a triode-type electrophoretic display in accordance with an exemplary embodiment of the present invention.

FIG. 2 is a fragmental cross-sectional view of the faceplate portion of the device shown in FIG. 1 taken along section line II-II and looking in the direction of the arrows.

FIG. 3 is a fragmental cross-sectional view of a faceplate like that shown in FIG. 2, but in accordance with a prior configuration as shown, e.g., in U.S. Patent No. 4,772,820.

FIG. 4 is a plan view of a mask for effecting the tapering grid insulator shown in FIG. 2.

FIG. 5 is a diagrammatic depiction of a step in the photoetching operation utilizing the mask shown in FIG. 4 to form the tapering grid insulator shown in FIG. 2.

### Best Mode for Carrying Out the Invention

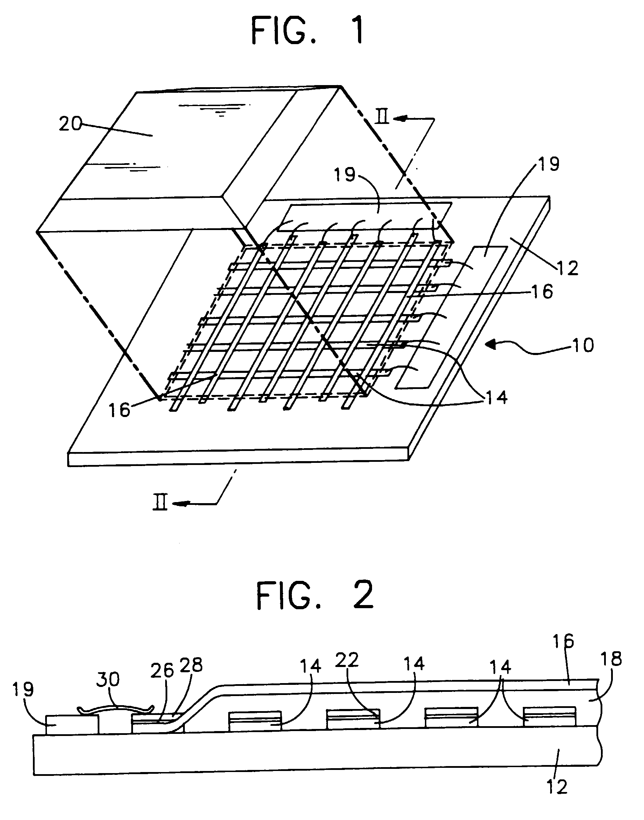

FIG. 1 shows the rear side of an electrophoretic display panel 10 as exemplified, e.g., by U.S. Patent No. 4,742,345 to Di Santo et al., this patent showing the general construction and components of an electrophoretic display panel. The panel 10 includes a faceplate 12, typically formed from glass, which serves as a substrate upon which is deposited a plurality of independent, electrically conductive cathode members 14 (horizontal rows) using conventional deposition and etching techniques. It is preferred that the cathode members 14 be composed of Indium Tin Oxide (ITO) as set forth in U.S. Patent No. 4,742,345. A plurality of independent grid conductor members 16 are superimposed in the horizontal over the cathode members 14 and are insulated therefrom by an interstitial photoresist layer 18 (see FIG. 2). The grid members 16 may be formed by coating the photoresist layer 18 with a metal, such as nickel, using sputtering techniques or the like and then selectively masking and etching to yield the intersecting but insulated configuration shown in FIGS. 1 and 2. Each cathode and grid member 14, 16 terminates at one end in a contact pad or is otherwise adapted to permit connection to display driver circuitry figuratively depicted as reference no. 19, as shall be more fully described below. In the embodiment shown, the representative driver circuit 19 is bonded to the faceplate 12 in accordance with the teachings of U.S. Patent No. 4,772,820. An actual display would utilize numerous such circuits as described in U.S. Patent No. 4,772,820. An anode cap 20 is sealably affixed to the faceplate 12 and over the cathode and grid members 14 and 16 to form an envelope for containing the dielectric fluid/pigment particle suspension. The anode cap 20 is formed from an insulating material, such as glass, and has an inner surface coating of conductor material to form the anode. Thus, by applying voltages to the cathode and grid members 14 and 16 and the anode 20, suspended pigment particles in the dielectric fluid can be made to accumulate near, or disperse from, the intersections of selected cathode and grid members 14 and 16 to translate these voltages into a visible display.

The discrete cathode and grid members 14 and 16 of the electrophoretic display 10 can assume a variety of voltages during operation for controlling the display operations of erase, hold and write at the numerous points of intersection defining a cathode/grid matrix. A workable panel would have a large number of intersections, e.g., 2,200 X 1,700 or a total of 3,740,000 separately addressable intersection points. For ease of illustration, however, a small set of discrete cathode and grid elements are shown in the figures. The dimensions of the respective elements have also been greatly enlarged for illustration and are not necessarily in proportion to an actual operational device. More verisimilar illustrations of electrophoretic displays, their components and electrical circuitry can be seen by referring to U.S. Patents Nos. 4,742,345 and 4,772,820, each being awarded to the inventors herein.

FIG. 2 shows the faceplate 12 of the electrophoretic display 10 of FIG. 1 in cross-section. The faceplate is preferably formed from glass and serves as a substrate upon which is deposited a plurality of independent electrically conductive cathode members 14. The layer of photoresist 18 insulates a plurality of grid elements 16 from the cathode elements. The photoresist 18 is etched down to the cathode members 14 to form strips of insulator which underlie the separate grid elements 16 by an operation set forth fully in U.S. Patent No. 4,742,345. In this view, four cathode elements 14 are visible and only a single grid element 16 is depicted. In order to facilitate connection of the cathode 14 and grid 16 elements of the display to display driver circuitry 19, the ends of the cathode members 14 are metalized, or coated with a metal or metals, in preparation for connection. FIG. 2 shows two metal layers 14 and 22. The first 14 is an ITO layer, and the second 22, a wire bonding layer of, e.g., chrome or aluminum. The grid members 16 are formed of a wire bonding metal, e.g., chrome or aluminum. A wire loop 30 electrically connects the grid element 16 to the device driver circuitry 19.

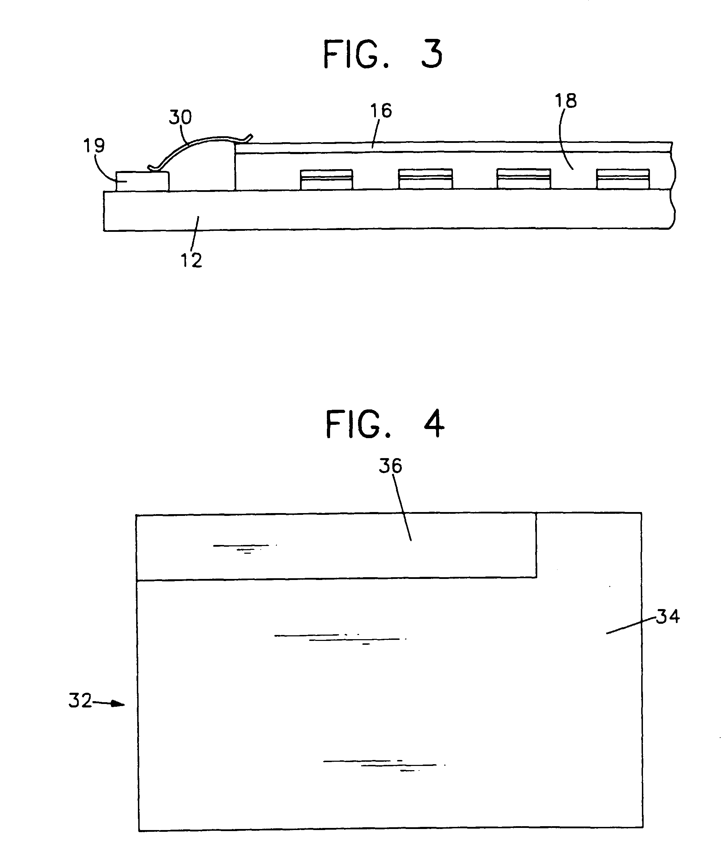

An inventive aspect of the present invention can be appreciated by comparing the insulator 18 and grid 16 configuration shown in FIG. 2 with that shown in FIG. 3 which depicts a photoresist layer 18 shape as was taught, e.g., in U.S. Patent No. 4,772,820. In FIG. 3, the insulator layer 18 comes to an abrupt end coterminal with the grid element 16. This results in a grid contact which is spaced an appreciable distance from the faceplate 12 and the device driver circuitry 19. Further, the grid element 16 is supported at the contact end by insulating photoresist material 18, which is deformable. When bonding is performed, the pressure exerted by the bonding machine to assist in affixing the wire loop 30 to the grid 16 is absorbed by a deformation of the insulator layer 18. Thus the bond is compromised and the process may have to be repeated by the bonding machine operator. Upon reexamining FIG. 2, it can be appreciated that the insulating photoresist layer 18 shown therein is sloped to permit the grid member 16 to descend from a plane above the faceplate 12 to the faceplate itself. This allows the contact end of the grid element 16 to reside on the hard glass of the faceplate 12, assuring a firm support for the contact when subjected to the pressure of the bonding machine and a good bond.

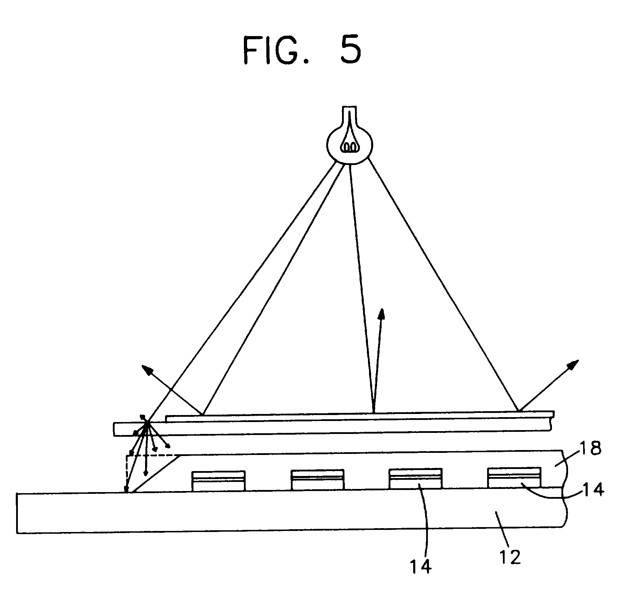

FIG. 4 shows a representative mask 32 which could be employed to create the tapered insulation layer 18 discussed above and shown in FIG. 2. The mask 32 is preferably made ftom glass and has one side partially coated with a reflective coating 34, e.g., chromium. The mask 32 shown is left uncoated along a portion 36 of one edge and in this area is clear. The clear portion 36 of the mask 32 will be positioned over that area of insulator 18 that is to be sloped down to the faceplate 12. The positioning of the mask 32 during the exposure of the photoresist 18 can be more easily visualized by examining FIG. 5.

FIG. 5 illustrates the selective and graduated removal of photoresist insulator 18 along one edge, by light exposure. Prior to performing the light exposure step illustrated, ITO cathode members 14 are formed on the faceplate 12 as described above. The photoresist insulator layer 18 is then applied by spin coating to a thickness of, e.g., 3.5 to 4.0 microns. If Shipley #1400-33 photoresist, manufactured by the Shipley Company, Inc. of Newton, Massachusetts is employed, a spinning rate of around 1,500 RPM will provide this thickness. This type of photoresist material is positive acting, i.e., that which is exposed to light is subject to removal by developer. The photoresist covered faceplate 12 is then pre-baked at 95 degrees Centigrade for 30 minutes. The coated faceplate 12 and mask 32 are then placed in a light exposure chamber. The mask 32 is positioned between the coated faceplate and light source at a distance therefrom to produce a graduated light intensity at a selected edge of the photoresist to render a sloping edge on the resist. The reflective coating 34 of the mask 32 points upwards towards the light and the clear portion 36 is positioned proximate the resist 18 that is to be removed and/or tapered. Upon exposure to light, those areas of photoresist 18 which are exposed to the strongest flux will be removed in their entirety and those areas having minimal exposure will be only slightly effected. At the edge of the reflective portion 34 of the mask 32, the light rays are refracted and dispersed upon passage through the clear portion 36. A range of light intensities is therefore produced upon the exposed resist 18, depending upon its position relative to the edge of the reflective layer 34. This situation is depicted in FIG. 5 by the vectors generated from a single ray of incident light passing through the clear area 36 proximate the reflective coating 34 edge. After the resist 18 is exposed, it is developed by developer. In order to provide a suitable substrate for depositing grid metal, the structure produced in the preceding steps must be thoroughly washed, especially in the area where resist has been removed. One method of accomplishing this is by rinsing the piece thoroughly in deionized water. The piece is then plasma etched in oxygen sufficiently to remove about 1,500 Angstroms from the surface. In a plasma etching machine, such as a Magastrip G^{•} manufactured by Drytek Co. of Wilmington, Massachusetts, modified for parallel plate etching, a time of one minute at a chamber pressure of 100 microns per square centimeter, a flow rate of 10 cubic centimeters per minute and a power density of about 1.6 watts per square centimeter is sufficient. The piece is then placed in an oven and baked at temperatures ramping up to 180 degrees Centigrade at 3 degrees Centigrade per minute. It is then baked at 180 degrees Centigrade for 90 minutes. The foregoing process yields a clean, flat photoresist insulator layer having a sloping edge where it was exposed to light through the clear portion of the mask. The individual grid members 16 are then formed on top of this layer by first sputtering on a layer of chrome or other suitable metal, such as nickel, about 3000 Angstroms thick. The chrome layer is covered by a layer of photoresist which is masked, exposed and developed and so on, all as described in U.S. Patent No. 4,742,345 pertaining to the formation of the individual grid elements. Thus, the insulation layer 18, having a configuration as shown in FIG. 2 is formed, permitting wire bonding to grid elements 16 that are supported at their contact end by the glass faceplate 12 rather than a photoresist layer 18.

It should be understood that the embodiments described herein are merely exemplary and that a person skilled in the art may make many variations and modifications without departing from the scope of the invention as defined in the appended claims.

## Claims

1. An electrophoretic display comprising:
(a) a fluid tight envelope having a planar portion (12) thereof which is at least partially transparent;
(b) an electrophoretic fluid contained within said envelope, said fluid having pigmented particles suspended therein;
(c) a plurality of elongated, substantially parallel first conductor members (14) deposited upon said planar portion and contained within said envelope;
(d) a plurality of elongated substantially coplanar second conductor members (16), said second conductor members extending in a direction perpendicular to said first conductor members wherein said first and second conductor members define a matrix with a plurality of intersections and wherein said first and second conductor members are selectively electrically chargeable to induce movement of said particles within said fluid, said particles being at least partially visible through said planar portion of said envelope; and
(e) a plurality of elongated grid insulator strips (18) interposed between and electrically insulating said first conductor members (14) from said second conductor members (16), each of said strips separating a respective first conductor member from the second conductor members by a selected distance,
characterized in that each of said insulator strips (18) has at its end portion a downwardly sloping edge surface and a base surface portion proximate said downwardly sloping edge surface such that the thickness of the strip is reduced towards its end, wherein said base surface is supported by said planar portion and a continuous surface portion of each second conductor member is in contact with and supported by both said downwardly sloping edge and said planar portion.

2. The device of Claim 1, wherein said envelope includes a cap (20) member having an end wall and side walls, said side walls defining a hollow within said cap member, said end wall coated with a conductor layer selectively chargeable to induce movement of said pigment particles, said side walls being sealingly affixed to said planar portion to form said fluid-tight envelope.

3. The device of Claim 2, wherein said electrophoretic display is a triode-type device, said conductor layer being the anode, said first members constituting the cathode and said second members constituting the grid.

4. The device of Claim 3, wherein said planar portion is a glass faceplate having a surface area larger than that covered by said cap, such that a border area of said faceplate extends beyond said cap and said fluid.

5. The device of Claim 4, wherein each of said plurality of first members and each of said plurality of second members have an end electrically connectable to an associated voltage source, said connectable end extending beyond said cap and said fluid.

6. The device of Claim 5, further including display driver circuitry (19) affixed to said faceplate on said border area, said circuitry connecting to said connectable ends of said second members and said first members.

7. The device of Claim 6, wherein said connectable ends are wire bonded to said display driver circuitry.

8. The device of Claim 7, wherein all said connectable ends are supported by said glass faceplate.

9. The device of Claim 8, wherein said connectable ends of said first members are metalized.

10. The device of Claim 9, wherein said connectable ends of said second members are metalized.

11. The device of Claim 10, wherein said insulator strips are photoresist.

12. A method for making an electrophoretic display having a glass faceplate (12) with a plurality of elongated substantially parallel first conductor members (14) deposited thereon, a plurality of elongated substantially parallel second conductor members (16), and a plurality of elongated insulator strips (18) interposed between and electrically insulating said first members from said second members, said strips affixed to said first members and to said second members and each of said strips supporting a corresponding second member thereon a selected distance from said first members and having at its end portion a downwardly sloping edge surface and a base surface portion proximate said downwardly sloping edge surface such that the thickness of the strip is reduced towards its end, wherein said base surface is supported by said faceplate (12) and a continuous surface portion of each second conductor member is in contact with and supported by said downwardly sloping edge and by said faceplate, comprising the steps of:
(a) forming said first members (14) upon a surface of said faceplate (12);
(b) applying a layer of photoresist to said surface;
(c) exposing said layer to light;
(d) simultaneously with said step of exposing, controlling the areas of said layer exposed to light with a mask (32), said mask having a portion thereof which partially and graduatedly shades a selected area of photoresist to produce a downwardly sloping edge thereon when developed;
(e) developing said exposed photoresist;
(f) applying a layer of conductor material onto said developed photoresist; and
(g) etching said conductor material and said underlying photoresist to form said second members (16) and said insulator strips (18) such that a continuous portion of each second member is disposed on a downwardly sloping edge surface defined of a corresponding insulator strip and on said faceplate.

13. The method of Claim 12, wherein said mask is formed from a transparent material partially coated with an opaque substance leaving a portion of said mask transparent to light and wherein said opaque coating is positioned toward the light during said simultaneous steps of exposing and controlling.

14. The method of Claim 13, wherein said mask is positioned relative to said light and said photoresist such that light passing through said transparent portion of said mask proximate said opaque coating is transmitted through said mask with a graduated flux intensity ranging from approximately zero to full luminous intensity, the intensity increasing with increasing distance from said opaque portion.

15. The method of Claim 14, wherein said graduated flux intensity is attributable to refraction and diffusion of light within said transparent portion of said mask.

16. The method of Claim 12, wherein said layer of photoresist is applied by spin coating and further including the step of pre-baking said faceplate and said photoresist after said step of applying said layer of photoresist.

17. The method of Claim 16 wherein said spin coating is performed at a spinning rate of approximately 1,500 RPM and said layer has a thickness of approximately 3.5 to 4.0 microns.

18. The method of Claim 16, wherein said step of pre-baking is at approximately 95 degrees Centigrade for approximately 30 minutes.

19. The method of Claim 12, further including the steps of washing and oxygen plasma etching said developed photoresist prior to said step of applying said layer of conductor material.

20. The method of Claim 19, wherein said step of washing is in deionized water and said step of plasma etching is performed for approximately 1 minute at a chamber pressure of about 100 microns per square centimeter, a flow rate of approximately 10 centimeters³ per minute and a power density of about 1.6 watts per centimeter².

21. The method of Claim 19, further including the step of baking said faceplate and said photoresist after said step of plasma etching.

22. The method of Claim 21, wherein said baking is at a temperature ramping up from room temperature to a temperature of about 180 degrees Centigrade at a ramping rate of 3 degrees Centigrade per minute, then baking at 180 degrees Centigrade for 90 minutes.

23. The method of Claim 14, wherein said layer of photresist is applied by spin coating, and further including the steps of pre-baking said faceplate and said photoresist after said step of applying said layer of photoresist, washing and oxygen plasma etching said developed photoresist prior to said step of applying said layer of conductor material, and baking said faceplate and said photoresist after said step of plasma etching.

24. The method of Claim 23, wherein said spin coating is performed at a spinning rate of approximately 1,500 RPM and said layer of photoresist has a thickness of approximately 3.5 to 4.0 microns, wherein said step of pre-baking is at approximately 95 degrees Centigrade for approximately 30 minutes, wherein said step of washing is in deionized water, wherein said step of plasma etching is performed for approximately 1 minute at a chamber pressure of about 100 microns per square centimeter, a flow rate of approximately 10 centimeters³ and a power density of about 1.6 watts per centimeter², and wherein said baking is at a temperature ramping up from room temperature to a temperature of about 180 degrees Centigrade at a ramping rate of 3 degrees Centigrade per minute, then baking at 180 degrees Centigrade for 90 minutes.

## Patentansprüche

1. Elektrophoretisches Display mit:
(a) einer flüssigkeitsdichten Umhüllung mit einem ebenen Abschnitt (12), der mindestens teilweise transparent ist;
(b) einem in besagter Umhüllung enthaltenen elektrophoretischen Fluid, in welchem pigmentierte Partikel schweben;
(c) einer Vielheit von länglichen, im wesentlichen ersten Leiterelementen (14), die auf besagtem ebenem Abschnitt abgelagert und in besagter Umhüllung enthalten sind;
(d) einer Vielheit von länglichen, im wesentlichen parallelen koplanaren zweiten Leiterelementen (16), die sich in einer senkrecht zu besagten ersten Leiterelementen liegenden Richtung erstrecken, wobei besagte erste und zweite Leiterelemente eine Matrix mit einer Vielheit von Schnittpunkten definieren und wobei besagte erste und zweite Leiterelemente selektiv elektrisch aufgeladen werden können, um eine Bewegung besagter Partikel in besagtem Fluid zu veranlassen, wobei besagte Partikel mindestens teilweise durch den ebenen Abschnitt besagter Umhüllung sichtbar sind; und
(e) einer Vielheit von länglichen Gitterisolationsstreifen (18), die zwischen besagten ersten Leiterelementen (14) angeordnet sind und diese von besagten zweiten Leiterelementen (16) isolieren, wobei jeder Streifen jeweils ein erstes Leiterelement um einen vorgegebenen Abstand von den zweiten Leiterelementen trennt, dadurch gekennzeichnet, daß besagte Isolationsstreifen (18) am Endabschnitt jeweils eine nach unten geneigte Randfläche und einen Grundflächenabschnitt neben besagter nach unten geneigter Randfläche haben, so daß die Dicke des Streifens in Richtung des Endes abnimmt, wobei besagte Grundfläche durch besagten ebenen Abschnitt gestützt wird und ein kontinuierlicher Flächenabschnitt jedes zweiten Leiterelements in Kontakt mit besagtem nach unten geneigtem Rand und besagtem ebenem Abschnitt ist und durch diese gestützt wird.

2. Vorrichtung nach Anspruch 1, wobei besagte Umhüllung ein Kappenelement (20) mit einer Endwandung und Seitenwandungen aufweist, besagte Seitenwandungen Höhlungen in besagtem Kappenelement bilden, besagte Endwandung mit einer Leiterschicht beschichtet ist, die zur Veranlassung der Bewegung besagter Pigmentpartikel selektiv aufgeladen werden kann und besagte Seitenwandungen zur Bildung besagter flüssigkeitsdichter Umhüllung abdichtend an besagtem ebenem Abschnitt befestigt sind.

3. Vorrichtung nach Anspruch 2, wobei besagtes elektrophoretisches Display eine Triodenvorrichtung ist, in der besagte Leiterschicht die Anode, besagte erste Elemente die Kathode und besagte zweite Elemente das Gitter bilden.

4. Vorrichtung nach Anspruch 3, wobei besagter ebener Abschnitt eine Glasfrontplatte mit einer Oberfläche ist, die größer ist als die von besagter Kappe abgedeckte Fläche, so daß sich ein Randbereich besagter Frontplatte über besagte Kappe und besagtes Fluid erstreckt.

5. Vorrichtung nach Anspruch 4, wobei besagte Vielheit der ersten Elemente und besagte Vielheit der zweiten Elemente jeweils ein Ende hat, das elektrisch mit einer zugehörigen Spannungsquelle verbunden werden kann, wobei sich besagtes verbindbares Ende über besagte Kappe und besagtes Fluid erstreckt.

6. Vorrichtung nach Anspruch 5, weiter mit einer auf besagter Frontplatte in besagtem Randbereich befestigten Displaytreiberschaltung (19), die an besagte verbindbare Enden besagter zweiter und erster Elemente angeschlossen wird.

7. Vorrichtung nach Anspruch 6, wobei besagte verbindbare Enden Drähte sind, die mit der Displaytreiberschaltung verbunden sind.

8. Vorrichtung nach Anspruch 7, wobei alle besagten verbindbaren Enden von besagter Glasfrontplatte getragen werden.

9. Vorrichtung nach Anspruch 8, wobei besagte verbindbare Enden besagter erster Elemente metallisiert sind.

10. Vorrichtung nach Anspruch 9, wobei besagte verbindbare Enden besagter zweiter Elemente metallisiert sind.

11. Vorrichtung nach Anspruch 10, wobei besagte Isolationsstreifen aus Photoresist bestehen.

12. Verfahren zur Herstellung eines elektrophoretischen Displays mit einer Glasfrontplatte (12) mit einer Vielheit von darauf aufgebrachten länglichen, im wesentlichen parallelen ersten Leiterelementen (14), einer Vielheit von länglichen, im wesentlichen parallelen zweiten Leiterelementen (16) und einer Vielheit von länglichen Isolationsstreifen (18), die zwischen besagten ersten Elementen angeordnet sind und diese von besagten zweiten Elementen isolieren, wobei besagte Streifen an besagten ersten und besagten zweiten Elementen befestigt sind, wobei jeder Streifen jeweils ein zweites Leiterelement in einem vorgegebenen Abstand von den ersten Leiterelementen stützt und wobei besagte Isolationsstreifen am Endabschnitt jeweils eine nach unten geneigte Randfläche und einen Grundflächenabschnitt neben besagter nach unten geneigter Randfläche haben, so daß die Dicke des Streifens in Richtung des Endes abnimmt, wobei besagte Grundfläche durch besagte Frontplatte (12) gestützt wird und ein kontinuierlicher Flächenabschnitt jedes zweiten Leiterelements in Kontakt mit besagtem nach unten geneigtem Rand und besagter Frontplatte ist und durch diese gestützt wird, bestehend aus den folgenden Schritten:
(a) Bildung besagter erster Elemente (14) auf einer Fläche besagter Frontplatte (12);
(b) Auftragen einer Schicht aus Photoresist (18) auf besagte Fläche;
(c) Belichten besagter Schicht;
(d) gleichzeitig mit dem Belichten Regeln der Flächen besagter belichteter Schicht mit einer Maske (32) mit einem Teil, der einen ausgewählten Bereich der Photoresistschicht zur Herstellung eines nach unten geneigten Randes bei der Entwicklung teil- und stufenweise beschattet;
(e) Entwickeln des besagten belichteten Photoresists;
(f) Aufbringen einer Schicht aus Leitermaterial auf besagten entwickelten Photoresist; und
(g) Ätzen besagten Leitermaterials und besagter darunterliegender Photoresistschicht zur Bildung besagter zweiter Elemente (16) und besagter Isolationsstreifen (18) derart, daß ein kontinuierlicher Abschnitt jedes zweiten Elements auf einer nach unten geneigten Randfläche angeordnet ist, die durch einen entsprechenden Isolationsstreifen definiert und auf besagter Frontplatte angeordnet ist.

13. Verfahren nach Anspruch 12, wobei besagte Maske aus einem transparenten Material besteht, das teilweise mit undurchsichtigen Substanz beschichtet ist, die einen Teil besagter Maske lichtdurchlässig läßt, und wobei besagte undurchsichtige Beschichtung während der besagten gleichzeitigen Belichtungs- und Regelschritte in Richtung des Lichtes liegt.

14. Verfahren nach Anspruch 13, wobei besagte Maske im Verhältnis zu besagtem Licht und besagtem Photoresist so angeordnet ist, daß das durch besagten transparenten Teil besagter Maske neben besagter undurchsichtiger Beschichtung gehende Licht durch besagte Maske mit einer abgestuften Stromintensität mit einem Bereich von ungefähr null bis zur vollen Leuchtkraft übertragen wird und die Intensität mit zunehmender Entfernung von besagtem undurchsichtigem Teil zunimmt.

15. Verfahren nach Anspruch 14, wobei besagte abgestufte Stromintensität der Brechung und Diffusion des Lichtes innerhalb des besagten transparenten Teils besagter Maske zu verdanken ist.

16. Verfahren nach Anspruch 12, wobei besagte Photoresistschicht im Schleuderverfahren aufgetragen wird, mit dem weiteren Schritt des Vorbrennens besagter Frontplatte und besagten Photoresists nach dem Auftragen der besagten Photoresistschicht.

17. Verfahren nach Anspruch 16, wobei besagtes Schleuderverfahren mit einer Schleuderdrehzahl von ungefähr 1500 U/min ausgeführt wird und besagte Schicht eine Dicke von ungefähr 3,5 bis 4,0 Mikron hat.

18. Verfahren nach Anspruch 16, wobei besagter Schritt des Vorbrennens bei ungefähr 95 Grad Celsius erfolgt und rund 30 Minuten dauert.

19. Verfahren nach Anspruch 12, mit den weiteren Schritten des Waschens und Plasmaätzens des besagten entwickelten Photoresists vor besagtem Schritt des Auftragens besagter Leitermetallschicht.

20. Verfahren nach Anspruch 19, wobei besagter Schritt des Waschens mit Deionat erfolgt und besagter Schritt des Plasmaätzens ungefähr 1 Minute dauert und bei einem Kammerdruck von rund 100 Mikron je Quadratzentimeter, einem Durchfluß von ungefähr 10 Kubikzentimeter pro Minute und einer Leistungsdichte von rund 1,6 Watt pro Quadratzentimeter erfolgt.

21. Verfahren nach Anspruch 19, mit dem weiteren Schritt des Brennens besagter Frontplatte und besagten Photoresists nach besagtem Schritt des Plasmaätzens.

22. Verfahren nach Anspruch 21, wobei besagtes Brennen bei einer Temperatur erfolgt, die um je 3 Grad Celsius pro Minute von normaler Zimmertemperatur auf rund 180 Grad Celsius ansteigt und anschließend für 90 Minuten auf 180 Grad Celsius gehalten wird.

23. Verfahren nach Anspruch 14, wobei besagte Photoresistschicht im Schleuderverfahren aufgetragen wird, mit den weiteren Schritten des Vorbrennens besagter Frontplatte und besagten Photoresists nach dem Auftragen der besagten Photoresistschicht, des Waschens und Plasmaätzens des besagten entwickelten Photoresists vor besagtem Schritt des Auftragens besagter Leitermetallschicht und des Brennens besagter Frontplatte und besagten Photoresists nach besagtem Schritt des Plasmaätzens.

24. Verfahren nach Anspruch 23, wobei besagtes Schleuderverfahren mit einer Schleuderdrehzahl von ungefähr 1500 U/min ausgeführt wird und besagte Schicht eine Dicke von ungefähr 3,5 bis 4,0 Mikron hat, wobei besagter Schritt des Vorbrennens bei ungefähr 95 Grad Celsius erfolgt und rund 30 Minuten dauert, wobei besagter Schritt des Waschens mit Deionat erfolgt, wobei besagter Schritt des Plasmaätzens ungefähr 1 Minute dauert und bei einem Kammerdruck von rund 100 Mikron je Quadratzentimeter, einem Durchfluß von ungefähr 10 Kubikzentimeter pro Minute und einer Leistungsdichte von rund 1,6 Watt pro Quadratzentimeter erfolgt, und wobei besagtes Brennen bei einer Temperatur erfolgt, die um je 3 Grad Celsius pro Minute von normaler Zimmertemperatur auf rund 180 Grad Celsius ansteigt und anschließend für 90 Minuten auf 180 Grad Celsius gehalten wird.

## Revendications

1. Un afficheur électrophorétique comprenant:
(a) une enceinte étanche au fluide ayant une portion plane (12) de celle-ci qui est au moins partiellement transparente;
(b) un fluide électrophorétique contenu dans ladite enceinte, ledit fluide ayant des particules pigmentées en suspension dans celui-ci;
(c) une pluralité de premiers éléments conducteurs allongés, considérablement parallèles (14) déposés sur ladite portion plane et contenus dans ladite enceinte;
(d) une pluralité de seconds éléments conducteurs allongés, considérablement coplanaires (16), lesdits seconds éléments conducteurs s'étendant dans une direction perpendiculaire auxdits premiers éléments conducteurs dans lequel lesdits premiers et seconds éléments conducteurs définissent une matrice avec une pluralité d'intersections et dans lequel lesdits premiers et seconds éléments sont sélectivement chargeables électriquement pour induire un déplacement desdites particules dans ledit fluide, lesdites particules étant au moins partiellement visibles à travers ladite portion plane de ladite enceinte; et
(e) une pluralité de bandes isolantes de grille allongées (18) interposées entre et isolant électriquement lesdits premiers éléments conducteurs (14) desdits seconds éléments conducteurs (16), chacune desdites bandes séparant un premier élément conducteur respectif des seconds éléments conducteurs d'une distance sélectionnée, caractérisée en ce que chacune desdites bandes isolantes (18) a à sa portion d'extrémité une surface de bord inclinée vers le bas et une portion de surface de base à proximité de ladite surface de bord inclinée vers le bas de sorte que l'épaisseur de la bande est réduite vers son extrémité, dans lequel ladite surface de base est supportée par ladite portion plane et une portion de surface continue de chaque second élément conducteur est en contact avec et supportée par à la fois ledit bord incliné vers le bas et ladite portion plane.

2. Le dispositif de la revendication 1, dans lequel ladite enceinte inclut un élément de couvercle (20) ayant une paroi d'extrémité et des parois latérales, lesdites parois latérales définissant une cavité dans ledit élément de couvercle, ladite paroi d'extrémité revêtue d'une couche conductrice chargeable sélectivement pour induire un déplacement desdites particules pigmentées, lesdites parois latérales étant fixées de manière étanche à ladite portion plane pour former ladite enceinte étanche au fluide.

3. Le dispositif de la revendication 2, dans lequel ledit afficheur électrophorétique est un dispositif de type triode, ladite couche conductrice étant l'anode, lesdits premiers éléments constituant la cathode et lesdits seconds éléments constituant la grille.

4. Le dispositif de la revendication 3, dans lequel ladite portion plane est une plaque de verre ayant une zone de surface plus grande que celle couverte par ledit couvercle, de sorte qu'une zone de bordure de ladite plaque s'étende au-delà dudit couvercle et dudit fluide.

5. Le dispositif de la revendication 4, dans lequel chacun de ladite pluralité de premiers éléments et chacun de ladite pluralité de seconds éléments ont une extrémité électriquement connectable à une source de tension associée, ladite extrémité connectable s'étendant au-delà dudit couvercle et dudit fluide.

6. Le dispositif de la revendication 5, incluant en outre un circuit de commande d'afficheur (19) fixé à ladite plaque sur ladite zone de bordure, ledit circuit étant relié auxdites extrémités connectables desdits seconds éléments et desdits premiers éléments.

7. Le dispositif de la revendication 6, dans lequel lesdites extrémités connectables sont liées par fil audit circuit de commande d'afficheur.

8. Le dispositif de la revendication 7, dans lequel toutes lesdites extrémités connectables sont supportées par ladite plaque de verre.

9. Le dispositif de la revendication 8, dans lequel lesdites extrémités connectables desdits premiers éléments sont métallisées.

10. Le dispositif de la revendication 9, dans lequel lesdites extrémités connectables desdits seconds éléments sont métallisées.

11. Le dispositif de la revendication 10, dans lequel lesdites bandes isolantes sont photorésistantes.

12. Un procédé pour réaliser un afficheur électrophorétique ayant une plaque de verre (12) avec une pluralité de premiers éléments conducteurs allongés, considérablement parallèles (14) déposés sur celle-ci, une pluralité de seconds éléments conducteurs allongés, considérablement parallèles (16), et une pluralité de bandes isolantes de grille allongées (18) interposées entre et isolant électriquement lesdits premiers éléments conducteurs desdits seconds éléments conducteurs, lesdites bandes fixées auxdits premiers éléments et auxdits seconds éléments et chacune desdites bandes supportant un second élément correspondant sur celle-ci à une distance sélectionnée desdits premiers éléments et ayant sur sa portion d'extrémité une surface de bord inclinée vers le bas et une portion de surface de base à proximité de ladite surface de bord inclinée vers le bas de sorte que l'épaisseur de la bande est réduite vers son extrémité, dans lequel ladite surface de base est supportée par ladite plaque (12) et une portion de surface continue de chaque second élément conducteur est en contact avec et supporté par à la fois ledit bord incliné vers le bas et ladite plaque, comprenant les étapes de:
(a) formation desdits premiers éléments (14) sur une surface de ladite plaque (12) ;
(b) application d'une couche de photorésistance (18) sur ladite surface;
(c) exposition de ladite couche à la lumière;
(d) simultanément avec ladite étape d'exposition, contrôle des zones de ladite couche exposée à la lumière avec un masque (32), ledit masque ayant une portion de celui-ci qui fait écran partiellement et graduellement à une zone sélectionnée de photorésistance pour produire un bord incliné vers le bas sur celle-ci après développement;
(e) développement de ladite photorésistance exposée;
(f) application d'une couche de matériau conducteur sur ladite photorésistance développée; et
(g) attaque dudit matériau conducteur et de ladite photorésistance sous-jacente pour former lesdits seconds membres (16) et lesdites bandes isolantes (18) de sorte qu'une portion continue de chaque second élément soit déposée sur une surface de bord inclinée vers le bas définie d'une bande isolante correspondante et de ladite plaque.

13. Le procédé de la revendication 12, dans lequel ledit masque est formé d'un matériau transparent partiellement revêtu d'une substance opaque laissant une portion dudit masque transparente à la lumière et dans lequel le revêtement opaque est positionné vers la lumière pendant lesdites étapes simultanées d'exposition et de contrôle.

14. Le procédé de la revendication 13, dans lequel ledit masque est positionné par rapport à ladite lumière et à ladite photorésistance de sorte que la lumière passant à travers ladite portion transparente dudit masque à proximité dudit revêtement opaque est transmise à travers ledit masque avec une intensité graduée du flux allant d'approximativement zéro à une totale intensité lumineuse, l'intensité augmentant avec la distance croissante de ladite portion opaque.

15. Le procédé de la revendication 14, dans lequel ladite intensité graduée du flux est attribuable à la réfraction et diffusion de la lumière dans ladite portion transparente dudit masque.

16. Le procédé de la revendication 12, dans lequel ladite couche de photorésistance est appliquée par centrifugation et incluant en outre l'étape de précuisson de ladite plaque et de ladite photorésistance après ladite étape d'application de ladite couche de photorésistance.

17. Le procédé de la revendication 16, dans lequel ladite application par centrifugation est réalisée à un taux de rotation d'approximativement 1500 t/min et ladite couche a une épaisseur située approximativement entre 3,5 et 4,0 microns.

18. Le procédé de la revendication 16, dans lequel ladite étape de précuisson est à approximativement 95 degrés centigrade pendant approximativement 30 minutes.

19. Le procédé de la revendication 12, incluant en outre les étapes de lavage et d'attaque au plasma d'oxygène de ladite photorésistance développée avant ladite étape d'application de ladite couche de matériau conducteur.

20. Le procédé de la revendication 19, dans lequel ladite étape de lavage est dans de l'eau déminéralisée et ladite étape d'attaque au plasma est effectuée pendant approximativement 1 minute à une pression de chambre d'environ 100 microns par centimètre carré, une charge volumique d'approximativement 10 centimètres³ par minute et une puissance volumique d'environ 1,6 watts par centimètre².

21. Le procédé de la revendication 19, incluant en outre l'étape de cuisson de ladite plaque et de ladite photorésistance après ladite étape d'attaque au plasma.

22. Le procédé de la revendication 21, dans lequel ladite cuisson est à une température croissante d'une température ambiante à une température d'environ 180 degrés centigrade à un taux croissant de 3 degrés centigrade par minute, puis une cuisson à 180 degrés centigrade pendant 90 minutes.

23. Le procédé de la revendication 14, dans lequel ladite couche de photorésistance est appliquée par centrifugation, et incluant en outre les étapes de précuisson de ladite plaque et de ladite photorésistance après ladite étape d'application de ladite couche de résistance, le lavage et l'attaque au plasma d'oxygène de ladite photorésistance développée avant ladite étape d'application de ladite couche de matériau conducteur, et cuisson de ladite plaque et de ladite photorésistance après ladite étape d'attaque au plasma.

24. Le procédé de la revendication 23, dans lequel ladite application par centrifugation est réalisée à un taux de rotation d'approximativement 1500 t/min et ladite couche a une épaisseur située approximativement entre 3,5 et 4,0 microns, dans lequel ladite étape de précuisson est à approximativement 95 degrés centigrade pendant approximativement 30 minutes, dans lequel ladite étape de lavage est dans de l'eau déminéralisée, dans lequel ladite étape d'attaque au plasma est effectuée pendant approximativement 1 minute à une pression de chambre d'environ 100 microns par centimètre carré, une charge volumique d'approximativement 10 centimètres³ et une puissance volumique d'environ 1,6 watts par centimètre², et dans lequel ladite cuisson est à une température croissante d'une température ambiante à une température d'environ 180 degrés centigrade à un taux croissant de 3 degrés centigrade par minute, puis une cuisson à 180 degrés centigrade pendant 90 minutes.
